# EUROPEAN PATENT APPLICATION

(11) **EP 4 721 702 A1**
(43) Date of publication of application: **08.04.2026**
(21) Application number: 24386116.8
(22) Date of filing: 07.10.2024
(51) Int. Cl.: A61F 2/16

(54) **AN ARTIFICIAL CAPSULAR BAG IMPLANT WITH A SUPPORT SYSTEM FOR USE IN APHAKIC EYES**

(71) Applicant: University of Crete/ Special Account for Research Funds of University of Crete (SARF UoC), 74150 Rethimnon (GR); Detorakis, T. Efstathios, 71201 Heraklion, Crete (GR)
(72) Inventor: Efstathios, Detorakis T., 71201 Crete (GR)

(57) **Abstract**

The invention relates to an artificial capsular bag for housing an intraocular lens (IOL) implant in aphakic eyes. The capsular bag features a discoid element with an internal space designed to receive the IOL. This element comprises an anterior surface with a central opening for IOL access, a posterior surface with multiple holes for viscoelastic fluid drainage, and a peripheral wall connecting these surfaces. Supporting elements are symmetrically positioned along the peripheral wall to secure the capsular bag within the eye chamber.

## Description

### TECHNICAL FIELD

The present disclosure relates to an artificial capsule bag implant having a support system for use in aphakic eyes.

### BACKGROUND

Loss of the lens of the eye (aphakia) with deficient anatomical support of the lens capsule and Zinn zonule is reported at a rate of 0.5% (1/200 cases) in modern adult cataract surgery, while it is also a common outcome in eye injuries, degenerative age conditions such as pseudoexfoliation syndrome (PEX), in situations with lens ectopic (ectopia lentis), such as Marfan syndrome, Weil-Marchesani or metabolic conditions such as homocystinuria. Aphakia is much more common in the case of cataract surgery or ocular trauma in the pediatric population, where a lens exchange may be required due to developmental changes in eye optics. The treatment of aphakia with aphakic glasses is generally troublesome due to the large thickness of the aphakic glasses and the inability to use aphakic glasses in case of unilateral aphakia. In the case of aphakic contact lenses there is a need for frequent replacements (more difficult in children or the elderly), exposure to the risk of allergies or infections and increased costs of use. Support of the intraocular lens in the eye in case of loss of capsule and Zinn fibers can be achieved by a variety of surgical techniques including intraocular lens placement in the anterior chamber, intraocular lens support with haptics on the anterior or posterior surface of the iris, also referred to as Artisan type intraocular lens implant, intraocular lens suturing in iris or sclera. The extrusion of the intraocular lens haptics through supporting holes in the sclera and their fixation has also been proposed, achieved by various techniques, such as the use of biological glue or the widening or deformation of their edges, known as the Yamane technique, while the use of an intraocular lens with specially designed haptics bearing flexible fixation anchors, the Carlevale type intraocular lens provides another option. In all of the above cases, surgical technique is generally difficult requiring high level of skill and associated with a significant risk of complications, including cystic macular oedema, intraocular hemorrhage, loss of corneal endothelial cells, retinal detachment and lens tilt. Moreover, the replacement of an intraocular lens with another of different refractive power is difficult in the present techniques of surgical fixation of intraocular lens implants for the correction of aphakia, accompanied by a significant risk of intraoperative and postoperative complications, despite the fact that the final position of the intraocular lens implant inside the eye, referred to as effective lens position (ELP), is not predictable and thus errors in the selected lens power may occur, resulting in the need for intraocular lens exchange. Finally, loss of internal compartmentalization of the eyeball in anterior and posterior segments occurs, resulting in the transfer of forces and biochemical mediators from one to the other with consequences for ocular biomechanical behavior and physiology.

In US 8,900,300 and US 9,763, 771 artificial capsular bag systems have been proposed but not for the treatment of aphakia but for maintaining the volume of the natural capsular sac, so that the lens maintains a stable position within it and the refractive effect remains stable. Capsule bags have also being proposed to act as carriers of electronic sensors, e.g. in EP4029474, or to support an unstable Zinn zone during cataract surgery.

Despite the advancements in this field, there remains a need for improved designs of artificial capsular bags for aphakic eyes that address the above challenges comprehensively.

### SUMMARY

The present invention aims to provide an innovative solution that overcomes the limitations of existing devices, offering enhanced stability, biocompatibility, and ease of use, thereby improving the outcomes for patients undergoing IOL implantation in aphakic eyes.

According to an aspect of the present invention, an artificial capsular bag for housing an intraocular lens (IOL) implant in aphakic eyes is presented. The capsular bag comprising:
a discoid element forming a housing with an internal space configured to receive the IOL, the discoid element comprising:
an anterior surface and a posterior surface positioned on opposing sides of the housing and connected via a peripheral wall, and
a supporting system configured to secure the discoid element in the eye chamber;
wherein the anterior surface comprising a central opening for accessing the internal space of the housing, and the posterior surface comprising a plurality of holes for the escape of viscoelastic fluid, and
wherein the supporting system comprises a plurality of supporting elements positioned at symmetrical locations along the peripheral wall.

The design of the artificial capsular bag as a transparent implant consisting of 2 layers corresponding to the natural anterior and posterior capsule with a hole in the anterior layer corresponding to size and shape in the hole of the anterior capsulorhexis performed during phacoemulsification surgery as well as holes for the escape of viscoelastic fluid, to prevent complications associated with viscoelastic fluid entrapment in the such as capsular block or elevated intraocular pressure. With the proposed artificial capsular bag implant, the original anatomy of the irido-lenticular area is restored by replacing the capsular bag and supporting fibers of the Zinn zone with the proposed artificial capsular bag and the supporting element, also referred to as anchoring system or supporting system, of either sutures or transcleral anchors, which transscleral anchors are also broadly referred to as anchoring elements.

The capsular bag implant houses the intraocular lens implant, being independent from it. This provides the capability of minimally invasive intraocular lens implant exchange in cases of errors in biometry, differences in the final intraocular lens implant position along the anterior-posterior axis (ELP) or developmental changes in refraction in the case of childhood aphakia. Moreover, it enables the use of any type of intraocular lens implant design, including all haptic system designs (one piece, three-piece or plate haptics and monofocal, trifocal, multifocal or extended depth of field optics).

According to embodiments of the present invention, the discoid element is made of a biocompatible elastic material such that the artificial capsular bag is deformable to enable insertion through a sutureless incision into the eye chamber. The use of a biocompatible elastic material makes the artificial capsular bag deformable, facilitating its insertion through a sutureless incision. This minimally invasive approach reduces surgical trauma, shortens recovery time, and lowers the risk of complications, making the procedure safer and more comfortable for patients.

According to embodiments of the present invention, the discoid element is made of a biocompatible transparent material such that the restoration of the refractive power of the eye depends on the power and the Effective Lens Position (ELP) of the intraocular lens implant. Using a biocompatible transparent material for the discoid element ensures that the optical clarity and refractive power of the eye are restored based on the IOL's power and Effective Lens Position (ELP). This transparency is crucial for optimal visual outcomes, as it allows light to pass through unobstructed, ensuring clear vision and accurate optical correction.

According to embodiments of the present invention, the discoid element is made of a material that is compatible with sterilization and intraocular implantation, such as silicone or acrylic-based polymers. The selection of materials compatible with sterilization and intraocular implantation, such as silicone or acrylic-based polymers; ensures that the artificial capsular bag can be safely sterilized and implanted without adverse reactions. These materials provide durability and biocompatibility, essential for long-term stability and safety of the implant, reducing the risk of infection and ensuring the longevity of the device.

According to embodiments of the present invention, the supporting elements are positioned at predetermined locations around the peripheral wall so as to maintain the capsular bag in a fixed position. The strategic placement of the supporting elements at predetermined locations around the peripheral wall, ensuring the artificial capsular bag remains securely in a fixed position within the eye chamber. This precise positioning enhances the stability of the capsular bag, preventing unwanted movement or rotation after implantation.

According to embodiments of the present invention, the anterior and posterior surfaces have a flat profile. The holes in the posterior surface being distributed so as to achieve optimum drainage of the viscoelastic fluid. The flat profile of the anterior and posterior surfaces of the artificial capsular bag enhances its stability and positioning within the eye chamber. This design minimizes potential distortions or irregularities that could affect the housing of the intraocular lens (IOL). By providing a consistent and stable surface, the flat profile ensures that the IOL maintains its intended Effective Lens Position (ELP), which is critical for achieving precise visual correction. Additionally, the flat surfaces simplify the insertion and positioning process during surgery, reducing the risk of complications and improving surgical efficiency. Furthermore, the distribution of holes in the posterior surface is optimized for effective drainage of viscoelastic fluid, preventing fluid buildup and potential increases in intraocular pressure. Overall, these design features contribute to more predictable and reliable outcomes, enhancing patient satisfaction with the visual results and overall surgical experience.

According to embodiments of the present invention, the supporting elements are in the form of supporting holes configured to receive fixating sutures for securing the discoid element in the eye chamber. The use of holes as supporting elements configured to receive fixating sutures, which offers enhanced fixation and stability of the artificial capsular bag within the eye chamber. This design allows for precise suture placement, ensuring the capsular bag remains securely in the desired position, preventing displacement or rotation. The holes provide flexibility during surgery, enabling the surgeon to adjust the tension and positioning for optimal placement, which is crucial for achieving the correct Effective Lens Position (ELP) of the intraocular lens (IOL) for proper visual correction. Additionally, this approach accommodates various surgical techniques, improving ease of use and expanding the applicability of the device.

According to embodiments of the present invention, the supporting elements are in the form of anchoring arms extending from the peripheral wall. For example the supporting system may comprise at least four anchoring arms, each extending from a quartile location on the peripheral wall. The anchoring arms are made of a biocompatible and semi-rigid material, such as polypropylene or nylon . The positioning of at least four anchoring arms at quartile intervals along the peripheral wall of the artificial capsular bag provides enhanced stability and uniform support within the eye chamber. This symmetrical arrangement ensures that the capsular bag is evenly secured, minimizing the risk of displacement or rotation after implantation. By distributing the anchoring points evenly, the capsular bag maintains the desired position more effectively, which is crucial for achieving and maintaining the correct Effective Lens Position (ELP) of the intraocular lens (IOL). This stability is essential for optimal visual outcomes, as it helps to maintain consistent optical performance of the IOL and reduces the likelihood of post-surgical complications. The use of biocompatible and semi-rigid materials, such as polypropylene or nylon, for the anchoring arms offer a balance between flexibility and rigidity, providing the necessary strength to securely anchor the artificial capsular bag within the eye chamber while allowing for some adaptability to the eye's natural movements. This semi-rigid characteristic ensures that the anchoring arms can maintain the capsular bag's stable position without causing undue stress or damage to the surrounding ocular tissues. Additionally, the biocompatibility of these materials ensures that they do not elicit adverse immune responses, thereby reducing the risk of inflammation or rejection. Overall, the use of these materials enhances the durability, safety, and effectiveness of the implant, leading to improved surgical outcomes and patient satisfaction.

According to an embodiment of the present invention, the supporting elements are in the form of handles provided at opposite locations along the peripheral wall. Each handle comprises one or more supporting holes for the placement of fixating sutures. The inclusion of handles, also referred to as side arms, at opposite locations along the peripheral wall provides a convenient means for handling and positioning the artificial capsular bag during implantation. These handles, also referred to as side arms, facilitate easier manipulation by the surgeon, improving precision and control during the placement of the capsular bag within the eye chamber. This design feature enhances the stability and accuracy of the implantation process, ensuring the intraocular lens (IOL) is correctly positioned for optimal visual outcomes. The holes allow for the sutures to be precisely placed through the handles, anchoring the capsular bag firmly in the desired position within the eye chamber, which enhances the overall stability of the capsular bag, reducing the risk of displacement or rotation post-implantation.

According to embodiments of the present invention, each handle comprises at least one anchoring element. The anchoring elements enhance the stability and fixation of the capsular bag, ensuring that it remains firmly in place after implantation. The added stability provided by the anchoring elements helps to maintain the correct Effective Lens Position (ELP) of the intraocular lens (IOL), which is essential for achieving optimal visual correction and preventing post-operative complications

According to embodiments of the present invention, the handles are positioned at an angle with respect to at least the posterior surface. The handles being an integral part of the peripheral wall of the discoid element. Positioning the handles, also referred to as side arms, at an angle with respect to the anterior and posterior surfaces and provide them as integrated part of the peripheral wall, enhance the functionality and stability of the artificial capsular bag. The angled handles facilitate easier manipulation and precise positioning during surgery, ensuring correct alignment of the intraocular lens (IOL) for optimal visual outcomes. The integral design ensures robust structural integrity, eliminating risks of detachment and enhancing overall stability within the eye chamber. This design improves surgical efficiency, reduces implantation complexity, and ensures consistent performance, leading to better patient outcomes and satisfaction.

In general, the support of the artificial capsular bag at two or more symmetrical locations along the peripheral wall, e.g. each positioned at a quadrant of the discoid element, with corresponding mounting anchors or sutures, maintains the capsular bag at a fixed position within the eye chamber parallel to the iris plane, thereby preventing the oblique position of the implant (tilt

### BRIEF DESCRIPTION OF THE DRAWINGS

The following drawings are provided as an example to explain further and describe various aspects of the invention.
Figures 1A to 1C show respectively an anterior oblique view, an anterior view, and a side view of an artificial capsular bag according to a first embodiment of the present invention.
Figures 1D and 1E show indicative dimensions of the artificial capsular bag according to the first embodiment of the present invention.
Figures 2A to 2D show respectively an anterior oblique view, an anterior view, a side view, and vertical oblique view of an artificial capsular bag according to a second embodiment of the present invention.
Figures 2E and 2F show indicative dimensions of the artificial capsular bag according to the second embodiment of the present invention.
Figures 3A to 3C show respectively an anterior oblique view, an anterior view, and a side view, of an artificial capsular bag according to a third embodiment of the present invention.
Figures 3D and 3E show indicative dimensions of the artificial capsular bag according to the third embodiment of the present invention.
Figures 4A to 4C show respectively an anterior oblique view, an anterior view, and a side view, of an artificial capsular bag according to a fourth embodiment of the present invention.
Figures 4D and 4E show indicative dimensions of the artificial capsular bag according to the fourth embodiment of the present invention.

### DETAILED DESCRIPTION

For the purposes of promoting an understanding of the principles of the present disclosure, reference will now be made to the implementations illustrated in the drawings, and specific language will be used to describe the same.

It will nevertheless be understood that no limitation of the scope of the disclosure is intended. Any alterations and further modifications to the described devices, methods and any further application of the principles of the present disclosure are fully contemplated as would normally occur to one skilled in the art to which the disclosure relates. In particular, it is fully contemplated that the features, components, and/or steps described with respect to one implementation may be combined with the features, components and/or steps described with respect to other implementations of the present disclosure.

In the detailed description and figures of this disclosure, reference numerals are used to denote corresponding elements that are similar in structure and function across multiple embodiments. It should be understood that when a reference numeral appears in different figures or embodiments, it refers to the same or substantially similar element unless otherwise specified. This convention is used to simplify the explanation and enhance the clarity of the various embodiments presented.

The presence of the lens within the eye is essential for focusing light on the retina. Loss of the lens (aphakia) is not a rare condition after eye injuries or complicated eye surgeries. It may also be a congenital or degenerative condition. The treatment of aphakia is technically difficult when the anatomical support of the lens (Zinn zonule and posterior capsule) has been lost and is currently performed either by conservative means (use of glasses or aphakic contact lens) or by surgical placement of an intraocular lens implant. In the first case, drawbacks are the increased thickness of aphakic glasses, the cost from the need for continuous contact lens replacement and the risk of infections or allergies. In the second case, support with sutures or haptics of the intraocular lens is required, with the risk, among others, of inflammation, bleeding, cystic macular edema, oblique position of the intraocular lens (tilt) or vibrations of the intraocular lens (pseudophakodonesis). The treatment of aphakia in the pediatric population is particularly challenging, since there is a risk of deficient vision development (amblyopia), the use of contact lenses is more difficult and frequent changes in the administered aphakic correction may be required, due to developmental changes of the eye. This invention proposes to support an intraocular lens in the posterior chamber of the eye using an artificial capsular bag which is fixed by an anchor system to the sclera, making it easy and quick to place it parallel to the iridial plane, through a small sutureless incision. Moreover, the risk of oblique position of the intraocular lens inside the artificial sac is minimized by the proposed implant and easy exchange of the intraocular lens in case of change in refractive status (pediatric aphakia) or errors in the calculation of the refractive power of the intraocular lens is possible. In addition, correction in the position of intraocular lenses with astigmatic correction (toric intraocular lenses) is possible, since a toric intraocular lens can be rotated within the artificial capsular sac until it takes the correct position according to astigmatic axis. Importantly, any type of intraocular lens design can be inserted in the proposed artificial capsular bag implant, including all haptic designs as well as monofocal, multifocal, trifocal or extended depth of field optics designs. Finally, the proposed patent restores the internal compartmentalization of the eyeball into anterior and posterior segments, which can have a significant effect on the biomechanical behavior of the eyeball.

It is hereby proposed to use an artificial capsular bag implant to receive the intraocular lens implant for the correction of aphakia. This artificial capsular bag implant can be inserted in the posterior chamber of the eye through a small sutureless incision, placed on the superior corneoscleral limbus. The proposed artificial capsular bag implant can be manufactured of biocompatible elastic and completely transparent material (such as acrylic polymer or silicone polymer or other similar material), and can have dimensions similar to the natural capsular bag and natural human lens, say a diameter of 8-10mm and say a total thickness of 4-5mm, with an internal space for the placement of the intraocular lens implant of say 3-4mm width. The proposed artificial capsular bag implant is a hollow discoid structure with an anterior surface with a circular opening, and a posterior surface with peripheral holes of smaller diameter, e.g. 0.5-1.0mm, for the exit from it of viscoelastic residues that are injected into the bag for the smooth insertion of the intraocular lens. The circular opening hole to its anterior surface has the same size, e.g. 5-7mm, as the anterior capsulorhexis opening performed to the anterior lens capsule in phacoemulsification surgery. The posterior surface of the implant has no refractive power, being flat and completely transparent, so that the restoration of the refractive power of the eye depends only on the power and the ELP of the intra-ocular lens implant. The thickness of the implant is such to enable deformation and insertion (folded) through a small sutureless incision. The internal space of the implant (i.e. the space between the anterior and posterior surfaces of the implant) has enough width to accommodate and stabilize one intraocular lens implant, e.g. 2-4mm.

The main advantage of the proposed invention is the technically easy placement of the artificial capsular sac with minimal manipulation in the intraocular environment and consequently minimal injury to intraocular structures such as (especially) corneal endothelial cells. Existing intraocular lens support options, either at the angle of the anterior chamber, iris or sclera, require intraocular manipulations (such as peripheral iridectomy and rotation of the intraocular lens implant in the anterior chamber often in contact with the anterior chamber angle) that can cause complications such as bleeding, retinal or choroid detachment, intraocular inflammation or macular cystic edema, damage to the infiltrative filter with a subsequent increase in intraocular pressure or loss of corneal endothelial cells with subsequent corneal clouding. With the proposed artificial capsule sac, intraocular manipulations are minimized and limited to needle extrusion to exit the sutures from the points of entry adjacent to the corneoscleral limbus, whereas the artificial capsular bag implant itself is inserted directly with minimal manipulations into the posterior chamber of the eye through a sutureless superior corneal incision.

Another point of originality is the support of the artificial capsular bag in 4 quadrants with corresponding mounting anchors or sutures, according to embodiments of the present invention instead of the more commonly mentioned 2 attachment points of support of aphakic intraocular lenses. The 4-point support design prevents the oblique position of the implant (tilt) because it keeps the position of the implant parallel to the iris plane. According to embodiments of the present invention, the supporting system may comprise two anchoring haptics for easier implantation (similar to those of the Carlevale type lens) but rotation or tilt of the intraocular lens implant are prevented due to the presence of the side haptic loops. Providing a supporting system with a plurality of anchoring elements, or other type of supporting elements, prevents the oblique (tilted) intraocular lens position, which is relatively common with existing intraocular lens support techniques because these are usually based on two support points of the haptics of the intraocular lens, so the intraocular lens can rotate on the axis defined by these points and end-up in an oblique plane relative to the plane of the iris. On the contrary, in the case of the proposed invention, support is provided at multiple points equidistant from the corneoscleral limit, maximizing the possibility of parallel position of the intraocular lens with the level of the iris.

Another point of originality is the fact that the capsular bag implant houses the intraocular lens implant, being independent from it. This provides the capability of minimally invasive intraocular lens implant exchange in cases of errors in biometry, differences in the final intraocular lens implant position along the anterior-posterior axis (ELP) or developmental changes in refraction in the case of childhood aphakia. Moreover, it enables the use of any type of intraocular lens implant design, including all haptic system designs (one piece, three-piece or plate haptics and monofocal, trifocal, multifocal or extended depth of field optics). Furthermore, having the IOL implant being independent from the capsular bag has the advantage of easy exchange of the intraocular lens, in case of an unacceptable refractive result or in case the refractive state of the eye changes over time. This mainly applies to the pediatric population since developmental changes of the eyeball may be accompanied by refractive changes. A lens exchange is expected to be much easier in the case of the artificial sac compared to an intraocular lens implant placed in the natural bag, since adhesions develop over time between the intraocular lens implant and the walls of the natural capsular bag, which makes it difficult to mobilize and remove the intraocular lens implant at a later time. Moreover, having the IOL being independent from the capsular bag provides the possibility of rotating the intraocular lens implant within the artificial capsular bag, in case of using an intraocular lens with astigmatic correction, i.e. different refractive power per meridian (toric lens). In this case, if the initial placement is not completely accurate, it may be necessary to rotate the intraocular lens within the capsular bag at a later time in order to obtain the correct meridian orientation, which in the case of the natural capsular bag may be difficult due to adhesions with the walls of the sac.

Another important advantage of the proposed patent, which significantly differentiates it from the hitherto available techniques of aphakia correction, is the restoration of the internal architecture of the eyeball by inserting into the eyeball not only the lens but also its artificial capsular bag. The restoration of a larger part of the irido-lenticular septum allows the anatomical separation of the anterior from the posterior segments of the eyeball and the limitation of force transfer, biochemical mediators of inflammation and materials such as silicone oil in eyes with previous vitrectomy surgery between the posterior and anterior segments of the eyeball. More importantly, the independency of the proposed artificial capsular bag implant from any intraocular lens implant implies that any intraocular implant haptic or optic design (including one, three-piece or plate haptics and monofocal, trifocal, multifocal or extended depth optics) may be used, which increases the options of the surgeon in terms of intraocular lens implant selection, based on the individualized needs of patients.

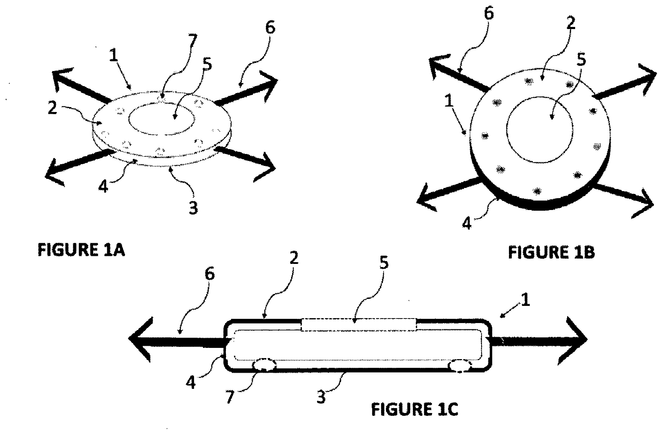

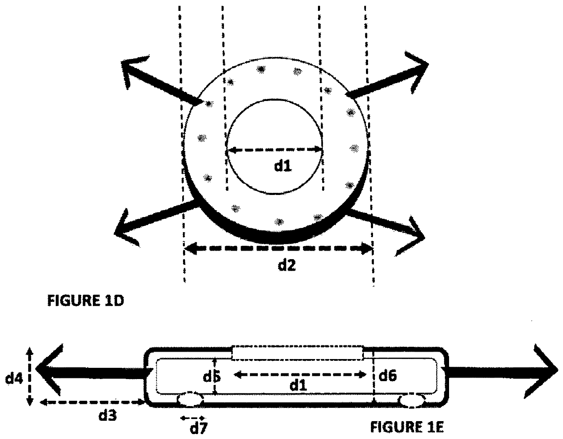

Figures 1A to 1C show different views of an exemplified embodiment of capsular bag implant according to the present invention. The presented capsular bag implant comprises a discoid element 1 having an anterior surface 2 and a posterior surface 3 connected via peripheral wall 4. The discoid element forms a housing for receiving via an opening 5 provided on the anterior surface an Intraocular lens (IOL) implant. The posterior surface is provided with a plurality of holes 7 distributed in a predetermined pattern, e.g along the periphery of the posterior surface 3, to allow for the escape of viscoelastic fluid. The posterior surface 3 is continuous centrally. The central opening 5 of the anterior surface 2 is dimensioned comparable to the dimensions of anterior capsulorhexis performed during phacoemulcification surgery. A supporting system comprising four anchoring elements 6 is provided for securing the discoid element 1 in eye chamber. The anchoring elements 6 are provided with arrow-like ends, and are distributed symmetrically along the 4 quartiles of the discoid element, for example at 10, 2, 4 and 8 o'clock positions. The anchoring elements 6 are oriented parallel or at slight angulation to the posterior surface of the artificial capsular bag. The anchoring elements 6, also referred to as anchor arms, support the capsular bag in the posterior chamber of the eye for capture through sclerotomy and easy extrusion of the anchoring elements 6 on the sclera, similar to the design of a Carlevale type intraocular lens. The discoid element 1 is constructed of bio-compatible elastic and transparent material with properties compatible with sterilization and intra-ocular implantation, such as silicone or acrylic polymers, or similar material. The anchoring elements 6 are constructed of bio-compatible semi-rigid material such as polypropylene or nylon. Figures 1D and 1E show indicative dimensions of the proposed implant of the capsular bag implant presented in Figures 1A to 1C.

Exemplified dimensions of the capsular bag of Figure 1A to 1C are shown in the figures 1D and 1E. The dimensions of the opening 5, indicated by d1, may be in the range of 6-8mm, the width of the discoid element 1, indicated by d2, may be in the range of 8-10mm, the length of the anchoring elements 6, indicated by d3, may be in the range of 2-3 mm, the height anchoring elements, indicated by d4, may be in the range of 1-2 mm, the height of the peripheral wall 4, indicated by d5, may be in the range of 2-4, the height of the discoid element, indicated by d6, may be in the range of 4-5mm, the width of the holes provided in the posterior surface, indicated by d7, may be -0.5mm.

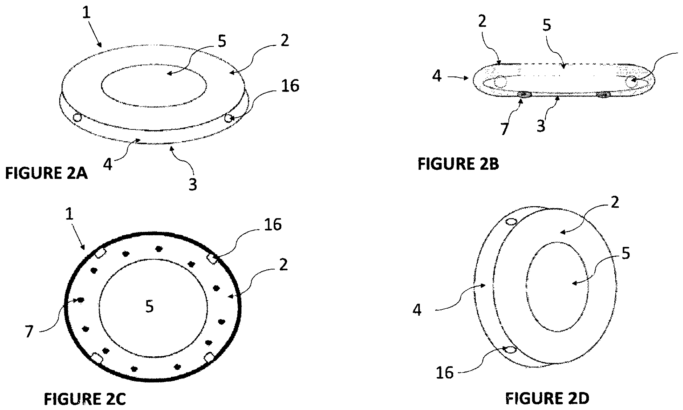

Figures 2A to 2D show different views of an exemplified embodiment of capsular bag implant according to the present invention. The presented capsular bag implant is similar to the capsular bag implant of figures 1A to 1C, with the main difference that the supporting system is the form of supporting holes 16 positioned symmetrically along the peripheral wall 4. These supporting holes 16 are used for the placement of fixating sutures. For example the supporting holes 16 may be positioned at quartile location of the discoid element 1. Similarly to figure 1, the discoid element 1 is constructed of bio-compatible elastic and transparent material with properties compatible with sterilization and intra-ocular implantation, such as silicone or acrylic polymers.

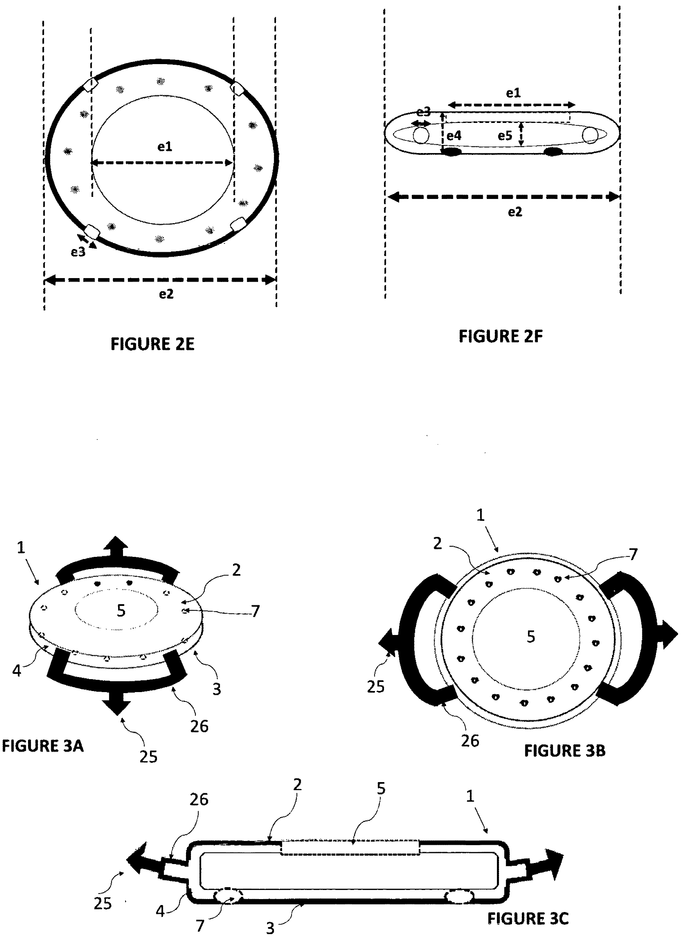

Figures 2E and 2F show indicative dimensions of the capsular bag of figure 2. The width of the supporting holes 16, indicated by e3, is in the range of 0.5-1.0 mm. The remaining indicative dimensions are the similar to the corresponding dimensions of figure 1.

Figures 3A to 3D show different views of an exemplified embodiment of capsular bag implant according to the present invention. The presented capsular bag implant is similar to the capsular bag implant of figures 1 and 2, with the main difference that the supporting system is the form of handles 26 extending from opposing location of the peripheral wall 4. At the middle of the parallel part on each handle 26 there is an fish-hook like anchoring element 25 for transcleral fixation. Each handle 26, also referred to as side arm, comprises two vertically oriented part extending from the peripheral wall 4, connected by a supporting part positioned parallel to the periphery of the discoid element 1.

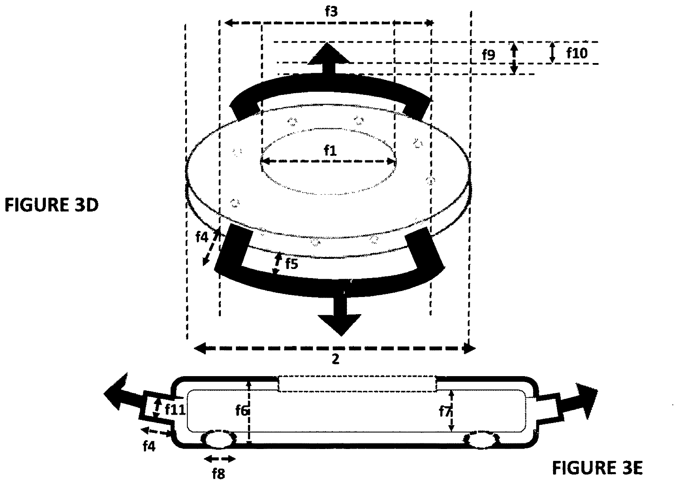

Figures 3D and 3E show indicative dimensions for the capsular bag implant of figure 3A to 3C. The length of the vertical connected parts, indicated by f4, is in the range of 1-2mm, the distance between the parallel supporting part and the peripheral wall 4, indicated by f5, is in the range of 0.5-1.0mm. The thickness of handle, indicated by fl 1, is around 0.5mm. The length of the anchoring element 25, indicated by f9, is in the range of 1.0-1.5mm, while the height of the fish-hook like end, indicated by f10, is in the range of 0.5-1.0mm. The remaining dimensions are similar to the corresponding dimensions of the capsular bag shown in figures 1 and 2.

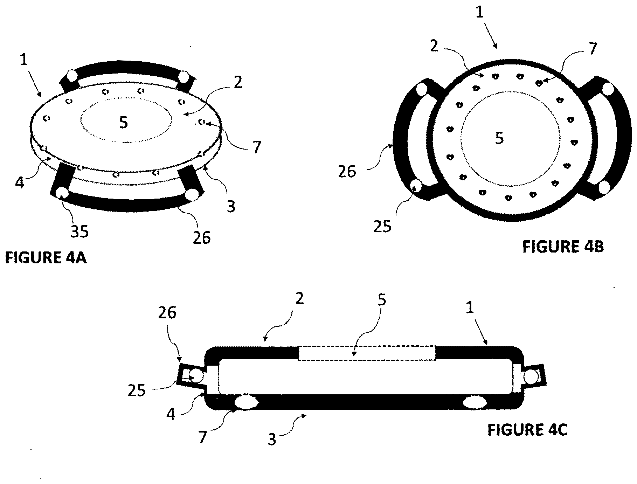

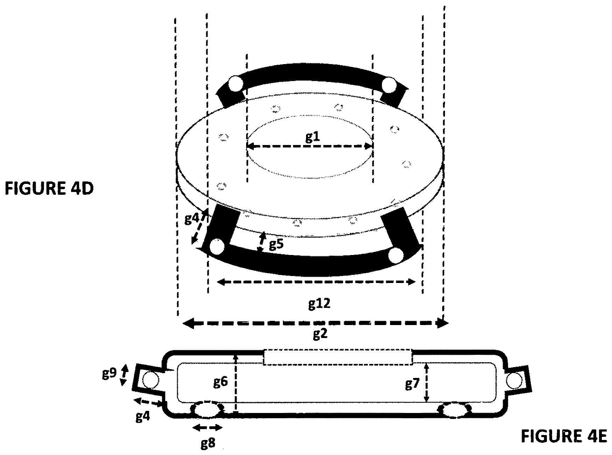

Figures 4A-4C show different views of an exemplified embodiment of capsular bag implant according to the present invention. The presented capsular bag implant is similar to the capsular bag implant of figure 4, with the main difference that the handle 26 comprises supporting holes 35, instead of the anchoring elements 25.

Indicative dimensions of the capsular bag of figure 4 are shown in figures 4D and 4E. The width of the handle, indicated by g12, is the range of 7-8mm, similar to the handle of the capsular bag of figure 3. The remaining dimensions are the same as the ones of the capsular bags presented in the previous figures.

The proposed artificial capsular bag implant, presented according to the exemplified embodiments shown in figures 1 to 4, is foldable so that it can be inserted via a small sutureless corneal or comeo-scleral incision, with a size preferably between about 1.5 mm and about 3 mm. Although an injector may be used for the insertion of the implant, it may also be manually folded and inserted into the eye with the use of adequate surgical forceps. Depending on the supporting system variation of the capsular bag implant prior to insertion certain surgical steps will have to be performed:
A) For the capsular bag implant presented in Figure 1, following a conjunctival peritomy, four partial thickness scleral flaps will be created at the corneoscleral area at 10 o'clock, 2 o'clock, 4 o'clock and 8 o'clock positions. Full thickness scleral incisions will be performed under the flaps. Following the insertion of the implant the anchoring arms will be passed transclerally and positioned under the partial thickness scleral flaps, either with microsurgical co-axial forceps or with sutures fed into the lumen of needles.
B) For the capsular bag implant presented in figure 2, two partial thickness scleral flaps will be created at the corneoscleral area at 3 o'clock and 9 o'clock positions. An adequate calibre (say 8.0 or 9.0) non-absorbable suture will be passed through the supporting holes on one side of the body of the artificial capsular bag implant and the procedure repeated for the fellow side of the implant. Full thickness scleral entrance with needle with adequate lumen (say 19-21G) will be performed, at 10 o'clock, 2 o'clock, 4 o'clock and 8 o'clock positions and the needles of the , also referred to as handles, will be fed into the lumen of the transcleral needles to assist in the transcleral passing of the non-absorbable side arm sutures. Following the insertion of the implant the sutures will be tied on both sides and the knots placed under the partial thickness scleral flaps at 3 o'clock and 9 o'clock positions.
C) For the capsular bag presented in figure 3, following a conjunctival peritomy, two partial thickness scleral flaps will be created at the corneoscleral area at 3 o'clock and 9 o'clock positions. Full thickness scleral incisions will be performed under the flaps. Following the insertion of the implant the anchoring arms will be passed transclerally and positioned under the partial thickness scleral flaps, either with microsurgical co-axial forceps or with sutures fed into the lumen of needles.
D) For the capsular bag presented in figure 4, following a conjunctival peritomy, two partial thickness scleral flaps will be created at the corneoscleral area at 3 o'clock and 9 o'clock positions. An adequate calibre (say 8.0 or 9.0) non-absorbable suture will be passed through the holes of the side arm of the artificial capsular bag implant and the procedure repeated for the fellow side of the implant. Full thickness scleral entrance with needle with adequate lumen (say 19-21G) will be performed, at 10 o'clock, 2 o'clock, 4 o'clock and 8 o'clock positions and the needles of the side arms, also referred to as handles, will be fed into the lumen of the transcleral needles to assist in the transcleral passing of the non-absorbable side arm sutures. Following the insertion of the implant the sutures will be tied on both sides and the knots placed under the partial thickness scleral flaps at 3 o'clock and 9 o'clock positions.

## Claims

1. An artificial capsular bag for housing an intraocular lens (IOL) implant in aphakic eyes, the capsular bag comprising:
a discoid element (1) forming a housing with an internal space configured to receive the IOL, the discoid element comprising:
an anterior surface (2) and a posterior surface (3) positioned on opposing sides of the housing and connected via a peripheral wall (4), and
a supporting system configured to secure the discoid element (1) in the eye chamber;
wherein the anterior surface (2) comprising a central opening (5) for accessing the internal space of the housing, and the posterior surface (2) comprising a plurality of holes (7) for the escape of viscoelastic fluid, and
wherein the supporting system comprises a plurality of supporting elements (6, 16, 25, 26, 35) positioned at symmetrical locations along the peripheral wall (4).

2. The artificial capsular bag of claim 1, wherein the discoid element (1) is made of a biocompatible elastic material such that the artificial capsular bag is deformable to enable insertion through a sutureless incision into the eye chamber.

3. The artificial capsular bag of claim 1 or 2, wherein the discoid element (1) is made of a biocompatible transparent material such that the restoration of the refractive power of the eye depends on the power and the Effective Lens Position (ELP) of the intraocular lens implant.

4. The artificial capsular bag of any one of the preceding claims, wherein the discoid element (1) is made of a material that is compatible with sterilization and intraocular implantation, such as silicone or acrylic-based polymers.

5. The artificial capsular bag of any one of the preceding claims, wherein the anterior and posterior surfaces (2, 3) have a flat profile.

6. The artificial capsular bag of any one of the preceding claims, wherein the supporting elements (6, 16, 25, 26, 35) are positioned at predetermined locations around the peripheral wall (4) so as to maintain the capsular bag in a fixed position.

7. The artificial capsular bag of any one of the preceding claims, wherein the supporting elements are in the form of supporting holes (16) configured to receive fixating sutures for securing the discoid element in the eye chamber.

8. The artificial capsular bag of any one of claims 1 to 6, wherein the supporting elements are in the form of anchoring arms (6) extending from the peripheral wall (4).

9. The artificial capsular bag of claim 8, wherein the supporting system comprises at least four anchoring arms (6), each positioned at a quartile of the peripheral wall (4).

10. The artificial capsular bag of claim 8 or 9, wherein the anchoring arms (6) are made of a biocompatible and semi-rigid material, such as polypropylene or nylon.

11. The artificial capsular bag of any one of claims 1 to 6, wherein the supporting elements are in the form of handles (26) provided at opposite locations along the peripheral wall.

12. The artificial capsular bag of claim 11, wherein each handle (26) comprises one or more supporting holes (35) for the placement of fixating sutures.

13. The artificial capsular bag of claim 11, wherein each handle (26) comprises at least one anchoring element (25) .

14. The artificial capsular bag of any one of claims 11 to 13, wherein the handles (26) are positioned at an angle with respect to at least the posterior surface (3).

15. The artificial capsular bag of any one of claims 11 to 14, wherein the handles (26) are an integral part of the peripheral wall of the discoid element.
